Europäisches Patentamt

European Patent Office

Office européen des brevets

(19)

(11) Publication number : **0 391 410 B1**

# (12) EUROPEAN PATENT SPECIFICATION

(45) Date of publication of patent specification :
28.07.93 Bulletin 93/30

(21) Application number : **90106509.4**

(22) Date of filing : **05.04.90**

(51) Int. Cl.⁵ : $A61K\ 31/715$

## (54) Antiviral composition.

(30) Priority : **06.04.89 JP 87295/89**

(43) Date of publication of application :
**10.10.90 Bulletin 90/41**

(45) Publication of the grant of the patent :
**28.07.93 Bulletin 93/30**

(84) Designated Contracting States :
**CH DE FR GB IT LI NL**

(56) References cited :
**EP-A- 0 240 098**
**EP-A- 0 246 654**
**CHEMICAL ABSTRACTS, vol. 98, no. 10, 7th March 1983, page 24, abstract no. 83293v, Columbus, Ohio, US; I. UMEZAWA et al.: "An acidic polysaccharide, Chlon A. form Chlorella pyrenoidosa. I. Physicochemical and biological properties", & CHEMOTHERAPY (TOKYO) 1982, 30(9), 1041-6**

(73) Proprietor : **DAIICHI PHARMACEUTICAL CO., LTD.**
**14-10, Nihonbashi 3-chome**
**Chuo-ku, Tokyo 103 (JP)**

(72) Inventor : **Nozaki, Junko**
**15-6-306, Shinjuku 6-chome, Shinjuku-ku Tokyo (JP)**
Inventor : **Takase, Hiroyuki, c/o Daiichi Pharmaceutical**
**Research Laboratories, 16-13, Kitakasai 1-chome**
**Edogawa-ku, Tokyo (JP)**

(74) Representative : **Kinzebach, Werner, Dr. et al Patentanwälte Reitstötter, Kinzebach und Partner Sternwartstrasse 4 Postfach 86 06 49 W-8000 München 86 (DE)**

## Description

This invention relates to the use of the sulfated polysaccharide DS-4152 or its derivatives wherein sodium of the sulfated polysaccharide DS-4152 is replaced by a pharmaceutical metal other than sodium, for preparing an antiviral composition.

The sulfated polysaccharide DS-4152 is a substance disclosed, for example, in European Patent Application No. 246,654, published November 25, 1987. The substance is known to have antitumor and vascularization inhibiting activities. However, the substance has not been previously known to have antiviral activity.

On the other hand, dextran sulfate, one of sulfated polysaccharides, is known to have antiviral activity (Proc. Nati. Acad. Sci. USA, 85 6132 (1988), Antimicrob. Agents Chemther. 32 1742 (1988)). From the clinical viewpoint, however, this substance is not yet fully satisfactory in that it can produce a serious adverse effect, namely potent anticoagulant effect.

EP-A-240 098 discloses compositions containing as active ingredient a natural or synthetic oligo- or polysaccharide having at least one S-oxo acid group attached to the saccharic carbon atom through a linking group of lower molecular weight. The compositions are described to be suitable for the treatment of diseases caused by retrovirus.

The present invention relates to the use of the sulfated polysaccharide DS-4152 or its derivatives wherein the sodium of DS-4152 is replaced by a pharmaceutical metal other than sodium for preparing an antiviral pharmaceutical composition.

DS-4152 is a sulfated polysaccharide species obtainable from the polysaccharide DF-4639 [cf. JP-A-56-67301 (the term "JP-A" as used herein means an "unexamined published Japanese Patent Application")], which can be isolated from a culture of the bacterial strain Arthrobacter sp. AT-25 (deposited with the Fermentation Research Institute, Agency of Industrial Science and Technology, Ministry of International Trade and Industry, Japan as Micrococcus sp. AT-25 with the accession number FERM P-5255, and deposited under Budapest Treaty as Arthrobacter sp. AT-25 with the accession number FERM BP-1357), by removing therefrom pyrogenic substances, which is contained therein and have a molecular weight of not less than about $15 \times 10^4$, using appropriate fractionation techniques, for example, gel filtration, ultrafiltration, dialysis and/or alcohol precipitation.

The sulfated polysaccharide DS-4152 has the following physicochemical characteristics:

(1) Molecular weight (according to gel filtration method):
   About $29,000 \pm 3,000$

(2) Elemental analysis (ranges for 5 lots):
   C, 24.42 to 25.76%; H, 3.34 to 3.98%; N, 0.51 to 0.89%; S, 10.6 to 11.7%; P, 0.77 to 1.06%.

(3) Sugar content and protein content:
   Sugar content (%): About $57 \pm 3$ (Phenolsulfuric acid method, with galactose as a standard)
   Protein content (%): $1 \pm 0.5$ (Lowry-Folin method, with bovine serum albumin as a standard)

(4) Specific rotation:
   $[\alpha]_D^{25}$ = About $-37° \pm 1°$ (0.5% aqueous solution)

(5) Main absorption bands in infrared absorption spectrum:
   1240, 840 (shoulder), 810 (cm$^{-1}$; KBr)

(6) Solubility:
   Freely soluble in water; practically insoluble in ethyl ether, benzene, chloroform, methanol, ethanol and other organic solvents.

(7) Color reactions:
   It can give positive phenol-sulfuric acid, anthronesulfuric acid, biuret and Lowry-Folin reactions. Its hydrolysate can give positive Elson-Morgan and nihydrin reactions. It can give negative carbazole and Sakaguchi reactions.

(8) Degree of basic, neutral or acidic:
   About pH 6 to 8 (3% aqueous solution)

(9) Constituent sugar, sulfate group and phosphorus content ratio:
   The molar ratio of D-Glucose/D-galactose/sulfate group as $SO_3Na$/P (D-glucose content being taken as 10) is about 10:61:73:6.

(10) Constituent amino acids and amino sugars:
   Analysis of its hydrolysate on an amino acid analyzer has revealed the presence of alanine, glycine, glutamic acid, diaminopimelic acid, glucosamine and muramic acid.

The derivative of DS-4152, wherein sodium of DS-4152 is replaced to another pharmaceutical metal, can be prepared by usual salt exchanging methods such as a method using a ion exchanging chromatography.

The above pharmaceutical metal other than sodium can include alkali metal such as potassium lichium, alkaline earth metal such as calcium, magnesium, and the like.

The composition according to the present invention is effective against RNA viruses, for example, retroviruses such as AIDS virus (human immunodeficiency virus; HIV), e.g., HIV-1 or HIV-2, and Human T cell Leukemia virus (HTLV), e.g., HTLV-I or HTLV-II, as well as DNA viruses, for example, viruses of Herpesviridae, such as cytomegalovirus and type I or type II herpes simplex virus, and type C, type B or type A hepatitis virus. Administration of DS-4152 or its derivatives can be expected to be particularly effective against HIV and viruses of Herpesviridae.

DS-4152 or its derivatives is a highly safe substance. Its acute intravascular toxicity ($LD_{50}$) in mice (slc-ddy strain, male, 6 weeks of age) is not less than 2,000 mg/kg.

DS-4152 or its derivatives, together with a conventional pharmaceutical carrier or excipient such as lactase, cornstarch, talc, magnesium stearate, can be made up into such dosage forms as solutions, powders, granules, tablets, or injectable solutions or suppository by conventional pharmaceutical techniques.

DS-4152 or its derivatives can be administered orally, intravenously or intradermally, preferably intravenously. Generally, the daily dose is within the range of 0.1 to 10 g per human adult in the case of oral administration and within the range of 10 to 500 mg per human adult in the case of intravenous administration.

In in vitro testing, DS-4152 did not affect the proliferation of host cells but did show a selective growth inhibiting effect on HIV and on viruses of Herpesviridae, specifically, type I or type II herpes simplex virus (HSV-1 or HSV-2). Therefore, DS-4152 or its derivatives can be said to be an excellent antiviral agent useful in the treatment of infectious diseases caused by various kinds of virus, for example, AIDS, AIDS-related complex such as disseminated histoplasmosis, bronchial and pulmonary candidiasis including pneumocystic pneumonia, non-Hadgkin's lymphoma, kaposis sarcoma and herpes infections, as well as in the prevention of development of viral diseases in carriers of relevant viruses.

The following examples are further illustrative of the present invention but by no means limitative of the scope thereof.

## EXAMPLE 1

### Anti-HIV activity

### Materials

(1) HIV-1, LAV1 strain, was used as the virus and CEM/C113 cells as the host cells.
(2) RPMI-1640 medium (manufactured by Gibco) supplemented with 10% inactivated fetal calf serum was used as the culture medium.

### Method

Host cells precultured for 3 days were challenged with HIV at an MOI (multiplicity of infection) of 0.01 to 0.05. After an absorption period of 90 minutes, the cells were suspended in the culture medium at a concentration of $5 \times 10^5$ cells/ml. A 0.5 ml portion of the suspension, 0.4 ml of the culture medium and 0.1 ml of each test substance solution were poured into each of several wells on a 24-well multiplate. Incubation was carried out in 5% $CO_2$-95% air.

Control wells contained 0.1 ml of physiological saline in lieu of the test substance solution.

Cells were counted at timed intervals and percent cell viability determination was performed by the Trypan blue exclusion technique. At the same time, HIV antigen positive cell percentage (IFA positive rate) determination was performed by the indirect immunofluorescent antibody (IFA) technique. The results thus obtained are shown below in Table 1.

## TABLE 1

| Item | Days after Infection | DS-4152 (μg/ml) Infected Cells | | | | | Non-infected Cells |
|---|---|---|---|---|---|---|---|
| | | 100 | 50 | 25 | 12.5 | 0 | |
| Cell count (Log·Cell/ml) | 4 | 6.32 | 6.31 | 6.25 | 6.24 | 6.05 | 6.47 |
| | 7 | 5.94 | 5.86 | 5.65 | 5.45 | 5.41 | 6.49 |
| Cell Viability (%) | 4 | 96.6 | 94.0 | 90.8 | 92.5 | 75.8 | 98.3 |
| | 7 | 82.1 | 71.3 | 66.7 | 44.4 | 29.8 | 97.8 |
| IFA Positive Rate (%) | 4 | 26.7 | 31.8 | 34.3 | 41.7 | 86.4 | 0 |
| | 7 | 31.3 | 59.3 | 55.8 | 64.3 | 80.0 | 0 |

As is evident from the data shown in Table 1, the cell viability increased and the IFA positive cell percentage decreased in a dose-dependent manner. The anti-HIV activity of DS-4152 was thus confirmed. Even when noninfected cells were treated with DS-4152 at a concentration of 100 μg/ml, any sign of cytotoxicity was not observed at all.

EXAMPLE 2

HIV reverse transcriptase inhibiting effect

Materials

(1) Reverse transcriptase of HIV:
CEM cells were inoculated with HIV by the method described in Example 1. Incubation was performed for 7 days and viral particles were purified from the culture supernatant by density gradient centrifugation. The purified viral particles were added to a final concentration of 0.1% aqueous solution of Triton[(R)] X-100, and the resultant mixture was used as an HIV reverse transcriptase preparation after allowing to stand at 37°C for 10 minutes.
(2) All substrates used in the enzymatic reaction system were purchased from Pharmacia except for the isotope-labeled thymidine triphosphate (hereinafter, TTP; manufactured by Amersham). Other reagents were purchased from Sigma.

Method

The HIV reverse transcriptase solution (10 μg protein) prepared as described above under Materials (1) and 1, 0.5.or 0.25 μg of the active ingredient (DS-4152) were added to the reaction system specified below and contained in a reaction tube (total amount 50 μg/tube), and the tube was kept at 37°C for 60 minutes. Reaction products resulting from the reverse transcriptase activity were quantitated by measuring the incorporation of isotope with a liquid scintillation counter. Distilled water was used as a control in lieu of the active ingredient.

Reaction system

50 mM Tris-HCl (pH 8.0)
5 mM Magnesium chloride
1 mM Dithiothreitol
20 mM Potassium chloride
10 μg/ml Poly(A)-oligo(dT)[(γA)$_n$(dT)$_{12-18}$]

10 nM Deoxythimidine triphosphate
$1.85 \cdot 10^4$ s$^{-1}$ (0.5 μCi) $^3$H-TTP
0.1% Triton$^{(R)}$ X-100
15% Glycerol

## TABLE 2

| DS-4152 (μg/ml) | Count (DPM) | Inhibition (%) |
|---|---|---|
| 0 (control) | 29,892 | 0 |
| 20 | 4,511 | 84.9 |
| 10 | 6,253 | 79.1 |
| 5 | 16,356 | 45.3 |

### Results

As evidenced by the data given in Table 2, it was confirmed that the active ingredient inhibits the reverse transcriptase activity of HIV in a dose-dependent manner.

### EXAMPLE 3

#### Effects on host cell surface HIV receptors

#### Materials

(1) CEM/C113 cells were used as the host cells.
(2) RPMI-1640 medium (Gibco) supplemented with 10% inactivated fetal calf serum was used as the culture medium.
(3) A monoclonal antibody to the OKT4A antigen was used as purchased from Orthomune.

#### Method

The active ingredient (DS-4152) was added to 1 ml of a host cell suspension ($2.5 \times 10^5$ cells/ml) to a concentration of 500 μg/ml and cultivation was performed in 5% $CO_2$-95% air for 48 hours. In a control run, the same volume of physiological saline was added and cultivation was carried out in the same manner. The cells were then stained with the FITC-conjugated anti-OKT4A antibody and the intensity of fluorescence was measured using a Showa Denko model CS-20 cell sorter. The results thus obtained are shown in Table 3.

## TABLE 3

| Group | Channel Number | Peak Channel Number |
|---|---|---|
| Control | 47 to 96 | 74 |
| DS-4152 | 26 to 59 | 43 |

As shown in Table 3, the DS-4152 treatment-free (control) cells, when treated with the FITC-conjugated anti-OKT4A antibody, gave a distribution with Channel No. 74 as a peak showing a high fluorescence intensity. On the contrary, the cells treated with DS-4152 in advance, when treated with the above-mentioned antibody, gave a distribution with Channel No. 43 as a peak showing a low fluorescence intensity. It was therefore presumed that DS-4152 covered the OKT4A antigen of the cultured cells and thereby inhibited the HIV-1 from being adsorbed on said antigen.

## EXAMPLE 4

### Anti-HSV activity

#### Materials

1) The KOS strain of type I herpes simplex virus (HSV-1) was used as the virus and Vero cells were used as the host cells.
2) MEM medium (manufactured by Eiken Kagaku) supplemented with 1.5% inactivated fetal calf serum was used as the culture medium.

#### Method

A host cell suspension was distributed into the wells of a 96-well plate and incubated at 37°C in 5% $CO_2$-95% air for 1 day. Active ingredient (DS-4152) solutions were added to respective wells, immediately followed by inoculation with the virus. Cultivation was performed for 72 hours under the same conditions as mentioned above.

In parallel, a viral infection-free system was provided for cytotoxicity evaluation. The other conditions were the same as mentioned above. Cells were fixed and stained, followed by absorbance measurement. The 50% cytopathic effect inhibition concentration ($CPIE_{50}$) was determined as an index of anti-HSV-1 activity and the 50% lethal concentration ($CLE_{50}$) as an index of cytotoxicity. The results obtained are shown in Table 4.

### TABLE 4

|  | $CPIE_{50}$ (µg/ml) | $CLE_{50}$ (µg/ml) |
|---|---|---|
| DS-4152 | 15.0 | >1000 |

As evidenced by the data given in Table 4, it was confirmed that DS-4152 does not exhibit toxicity to host cells but inhibits the proliferation of HSV-1 selectively.

## EXAMPLE 5

### In vitro anticoagulant activity

#### Materials

(1) Dextran sulfate sodium (manufactured by Seikagaku Kogyo; average molecular weight 10,700) was used as a positive control.
(2) Clotek (manufactured by Sanko Junyaku) was used for clotting or coagulation time (PCT) measurement.

#### Method

0.1 ml of saline solutions of the test substance in varied concentrations were respectively added to 0.1 ml portions of citrated rat serum. Each mixture was warmed at 37°C for 3 minutes and 0.1 ml of 0.025 M aqueous calcium chloride was then added to the mixture for causing coagulation to start. The clotting time was plotted against the concentration on an ordinary scale section paper, and the anticoagulant activity, which is defined as the concentration ($CT_2$) at which the clotting time is doubled as compared with that of the control group free of either test substance, was determined graphically. The results thus obtained are shown in Table 5.

## TABLE 5

| Test Substance | $CT_2$ ($\mu g/ml$) |
| --- | --- |
| DS-4152 | 23.5 |
| Dextran sulfate sodium | 11.3 |

It is clear from Table 5 that DS-4152 is weaker in anticoagulant activity than dextran sulfate sodium.

## Claims

1. The use of the sulfated polysaccharide DS-4152 or its derivatives wherein the sodium of said DS-4152 is replaced by a pharmaceutical metal other than sodium for preparing an antiviral pharmaceutical composition.

2. The use of claim 1, wherein the composition is effective against a human retrovirus, a human immunodeficiency virus, a virus of Herpesviridae and/or a type 1 herpes simplex virus.

## Patentansprüche

1. Verwendung des sulfatisierten Polysaccharids DS-4152 oder der Derivate davon, bei denen das Natrium des DS-4152 durch ein anderes pharmazeutisches Metall als Natrium ersetzt ist, zur Herstellung eines antiviralen pharmazeutischen Mittels.

2. Verwendung nach Anspruch 1, wobei das Mittel wirksam ist gegen ein Human-Retrovirus, ein Human-Immunodefizienz-Virus, ein Herpes viridae-Virus und/oder ein Herpes simplex-Virus vom Typ 1.

## Revendications

1. Utilisation du polysaccharide sulfaté DS-4152 ou de ses dérivés dans lesquels le sodium dudit DS-4152 est remplacé par un métal pharmaceutique autre que le sodium pour la préparation d'une composition pharmaceutique antivirale.

2. Utilisation selon la revendication 1, selon laquelle la composition est efficace vis-à-vis d'un rétrovirus humain, d'un virus du syndrome immunodéficitaire acquis, d'un virus de Herpesviridae et/ou d'un virus de l'herpès simplex du type 1.